# EUROPÄISCHE PATENTSCHRIFT

(11) **EP 2 544 685 B1**
(45) Veröffentlichungstag und Bekanntmachung des Hinweises auf die Patenterteilung: **11.05.2016**
(21) Anmeldenummer: 11730880.9
(22) Anmeldetag: 09.03.2011
(51) Int. Cl.: A61K 31/445, A61K 9/20, A61K 47/02

(54) **FESTE PHARMAZEUTISCHE ZUSAMMENSETZUNG, UMFASSEND DONEPEZIL-HYDROCHLORID DER KRISTALLINEN POLYMORPHEN FORM I**
SOLID PHARMACEUTICAL COMPOSITION, COMPRISING DONEPEZIL HYDROCHLORIDE OF THE CRYSTALLINE POLYMORPHOUS FORM I
COMPOSITION PHARMACEUTIQUE SOLIDE CONTENANT DE L'HYDROCHLORURE DE DONEPEZIL DE LA FORME 1 POLYMORPHE CRISTALLINE

(30) Priorität: 10.03.2010 DE 102010010998
(43) Veröffentlichungstag der Anmeldung: 16.01.2013
(73) Patentinhaber: STADA ARZNEIMITTEL AG, 61118 Bad Vilbel (DE)
(72) Erfinder: STUPAR, Biljana, 26300 Vrsac (RS); VUKOVIC , Stasa, 26300 Vrsac (RS); BOZIC , Jovana, 26300 Vrsac (RS)
(74) Vertreter: Kernebeck, Thomas
(86) Internationale Anmeldenummer: PCT/DE2011/000242
(87) Internationale Veröffentlichungsnummer: WO 2011/110166

(56) Entgegenhaltungen:
- WO-A1-2006/045512
- WO-A1-2008/012495
- US-A1- 2005 232 990

## Beschreibung

Die vorliegende Erfindung betrifft eine feste pharmazeutische Zusammensetzung, umfassend Donepezil-Hydrochlorid der kristallinen polymorphen Form I.

Donepezil ist der internationale Freiname (INN(*International Nonproprietary Name*)-Name) für 1-Benzyl-4-[(5,6-dimethoxy-1-indanon)-2-yl]methylpiperidin. Donepezil weist die folgende Strukturformel auf:

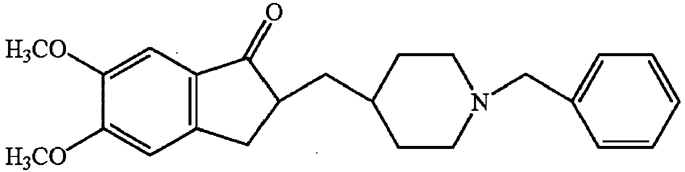

Donepezil ist ein Arzneistoff aus der Gruppe der Antidementiva und ist zur Behandlung der leichten bis mittelschweren Demenz vom Alzheimer-Typ zugelassen. Die Wirkung von Donepezil beruht auf der reversiblen Hemmung der Acetylcholinesterase.

In auf dem Markt befindlichen festen oralen Darreichungsformen wird Donepezil in Form von Donepezil-Hydrochlorid *(CAS(Chemical Abstracts Service)-*Nummer: 120011-70-3) eingesetzt. Auch in WO2006/045512A1, WO2008/012495A1 und US2005/0232990A1 werden feste orale Darreichungsformen enthaltend Donepezil-Hydrochlorid offenbart.

### BESTÄTIGUNGSKOPIE

Pharmazeutische Wirkstoffe können in mehr als nur einer festen Form existieren. Beispielsweise kann ein Wirkstoff in einer oder mehreren kristallinen Formen vorliegen und/oder in einer amorphen Form. Darüber hinaus kann ein Wirkstoff gelegentlich auch in einer oder mehreren solvatisierten Formen vorliegen. Häufig weisen verschiedene polymorphe Formen voneinander verschiedene physikalische und chemische Eigenschaften auf wie z.B. hinsichtlich der Löslichkeit und Hygroskopizität, oder zeichnen sich durch Eigenschaften aus, die eine verhältnismäßig einfache Formulierung des Wirkstoffs zu einer Darreichungsform ermöglichen. Bestimmte polymorphe Formen können ferner eine größere Stabilität als andere aufweisen, was etwa anhand einer größeren Tendenz einer bestimmten polymorphen Form zur spontanen Umwandlung in eine andere polymorphe Form gezeigt werden kann.

Von Donepezil-Hydrochlorid sind mehrere polymorphe Formen bekannt. WO 97/046527 A1 beispielsweise offenbart fünf kristalline polymorphe Formen des Donepezil-Hydrochlorids, die als Form I, II, III, IV bzw. Form V bezeichnet werden, sowie eine amorphe Form.

Donepezil-Hydrochlorid der kristallinen polymorphen Form I besitzt zwar gute pharmakologische Eigenschaften und wird daher auch in Darreichungsformen eingesetzt. Es hat aber den Nachteil, dass es sich verhältnismäßig schnell in andere polymorphe Formen umwandelt, insbesondere in die kristalline polymorphe Form III (vgl. WO 2006/090263 A1), weshalb bei auf Donepezil-Hydrochlorid der kristallinen polymorphen Form I basierenden Darreichungsformen eine gleichbleibende Arzneiformqualität nur für einen relativ kurzen Zeitraum gewährleistet werden kann.

Aufgabe der vorliegenden Erfindung ist es daher, eine feste pharmazeutische Zusammensetzung der eingangs genannten Art bereitzustellen, in welcher das Donepezil-Hydrochlorid der kristallinen polymorphen Form I über einen verhältnismäßig langen Zeitraum stabil ist.

Diese Aufgabe wird gelöst durch eine feste pharmazeutische Zusammensetzung, umfassend
a) kristallines Donepezil-Hydrochlorid mit einem unter Verwendung von Cu-K_{α1}-Strahlung erhaltenen Pulver-Röntgenbeugungsmuster, das die zehn intensitätsstärksten Signale bei den nachstehenden Beugungswinkeln 2θ mit der jeweilig zugeordneten relativen Intensität I/I₀ aufweist

| Beugungswinkel 2θ | relative Intensität I/I₀ |
|---|---|
| [°] | [%] |
| 4,91 | 48,43 |
| 12,64 | 100 |
| 13,07 | 58,57 |
| 13,84 | 22,05 |
| 16,07 | 35,24 |
| 16,87 | 26,21 |
| 21,16 | 71,72 |
| 23,00 | 24,30 |
| 23,90 | 33,38 |
| 24,17 | 20,49 |

b) ein Erdalkalimetallsalz der ortho-Phosphorsäure.

Überraschenderweise wurde festgestellt, dass Erdalkalimetallsalze der ortho-Phosphorsäure (H₃PO₄) die kristalline polymorphe Form I von Donepezil-Hydrochlorid stabilisieren. Entsprechend zeichnet sich die erfindungsgemäße feste pharmazeutische Zusammensetzung durch eine verhältnismäßig hohe Stabilität bezüglich der kristallinen polymorphen Form I von Donepezil-Hydrochlorid aus, weshalb die Arzneiformqualität der erfindungsgemäßen Zusammensetzung über einen relativ langen Zeitraum weitgehend gleichbleibend ist.

Unter dem Begriff "Stabilität" wird in diesem Zusammenhang die von der Arbeitsgemeinschaft für pharmazeutische Verfahrenstechnik (APV) erarbeitete Definition verstanden, nach welcher "Stabilität" die spezifikationsgerechte Qualität des Arzneimittels bis zum Ende der vom Hersteller festgelegten Laufzeit bedeutet. Die Qualität des Arzneimittels wird dabei durch den Wirkstoffgehalt und die Reinheit, die sensorisch wahrnehmbaren, physikalisch-chemischen und die mikrobiologischen Eigenschaften bestimmt, wobei der Wirkstoffgehalt bis zum Ende der Laufzeit 90 % des deklarierten Wertes nicht unterschreiten soll.

Im Rahmen der vorliegenden Erfindung ist unter dem Ausdruck "Donepezil-Hydrochlorid" das HCl-Additionssalz von Donepezil zu verstehen, wobei das Donepezil sowohl als R-Enantiomer, als S-Enantiomer als auch als R/S-Enantiomerengemisch vorliegen kann. Erfindungsgemäß bevorzugt ist es, dass das Donepezil als Racemat vorliegt.

In der erfindungsgemäßen Zusammensetzung ist Donepezil-Hydrochlorid der kristallinen polymorphen Form I enthalten. Donepezil-Hydrochlorid der kristallinen polymorphen Form I ist im Stand der Technik bekannt. In der kristallinen polymorphen Form I ist Donepezil-Hydrochlorid in hydratisierter Form enthalten, wobei der Wassergehalt variieren kann und beispielsweise 5 Gew.-% bis 6 Gew.-% beträgt (vgl. US 2008/0114173 A1). Erfindungsgemäß bevorzugt ist es, dass das in der erfindungsgemäßen Zusammensetzung enthaltene Donepezil-Hydrochlorid in Form des Monohydrates vorliegt. Der Wassergehalt des Wirkstoffs wird vorzugsweise nach Karl Fischer gemäß European Pharmacopoeia (Ph.Eur.) 5.7 Kapitel 2.5.12 bestimmt.

Die kristalline polymorphe Form I von Donepezil-Hydrochlorid weist ein unter Verwendung von Cu-K_{α1}-Strahlung erhaltenes Pulver-Röntgenbeugungsmuster auf, das die zehn intensitätsstärksten Signale bei den nachstehenden Beugungswinkeln 2θ mit der jeweilig zugeordneten relativen Intensität I/I₀ zeigt

| Beugungswinkel 2θ | relative Intensität I/I₀ |
|---|---|
| [°] | [%] |
| 4,91 | 48,43 |
| 12,64 | 100 |
| 13,07 | 58,57 |
| 13,84 | 22,05 |
| 16,07 | 35,24 |
| 16,87 | 26,21 |
| 21,16 | 71,72 |
| 23,00 | 24,30 |
| 23,90 | 33,38 |
| 24,17 | 20,49 |

wobei es in diesem Zusammenhang erfindungsgemäß bevorzugt ist, dass die entsprechende PXRD-Messung mittels eines Transmissionsdiffraktometer-Gerätes der Bezeichnung STADI P der Firma Stoe & Cie GmbH, Darmstadt, Deutschland, durchgeführt und die Geräte-/Messparameter wie folgt eingestellt werden:
- Wellenlänge: λ=0,154056 nm (Cu-K_{α1}-Strahlung)
- Monochromator: gebogen, Ge(111)
- Spannung: U=40 kV
- Stromstärke: I=30 mA
- Winkelbereich: 2θ=2° bis 40°
- Schrittweite: Δ 2θ=0,2°
- Messzeit: 20 s pro 0,2°-Schritt
- Detektor: linearer ortsempfindlicher Detektor (linear position sensitive detector (PSD))
- Probenträgermaske: Durchmesser: 8 mm

Die kristalline polymorphe Form I von Donepezil-Hydrochlorid weist darüber hinaus intensitätsschwächere Signale bei den nachstehenden Beugungswinkeln 2θ mit der jeweilig zugeordneten relativen Intensität I/I₀ auf

| Beugungswinkel 2θ | relative Intensität I/I₀ |
|---|---|
| [°] | [%] |
| 10,54 | 17,05 |
| 13,59 | 14,03 |
| 19,33 | 17,74 |
| 19,82 | 12,32 |
| 23,60 | 17,93 |
| 26,38 | 11,41 |
| 27,10 | 13,19 |
| 28,02 | 11,75 |
| 31,79 | 10,10 |

wobei es auch in diesem Zusammenhang bevorzugt ist, dass die entsprechende PXRD-Messung mittels des vorerwähnten Gerätes und bei den vorgenannten Geräte-/Messparametern durchgeführt wird.

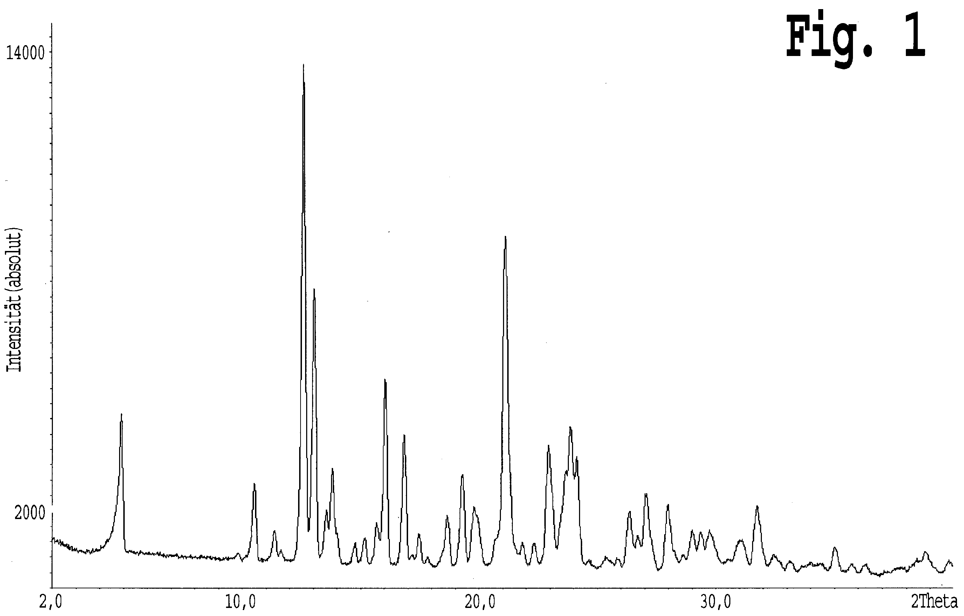

Gemäß einer besonders bevorzugten Ausführungsform der erfindungsgemäßen Zusammensetzung ist es vorgesehen, dass das in der Zusammensetzung enthaltene Donepezil-Hydrochlorid der kristallinen polymorphen Form I ein Donepezil-Hydrochlorid ist, dass bei einer Pulver-Röntgendiffraktometrischen-Vermessung, vorzugsweise mittels des vorerwähnten Gerätes und bei den vorstehend genannten Geräte- und Messparametern, das in der Fig. 1 gezeigte PXRD-Spektrum zeigt.

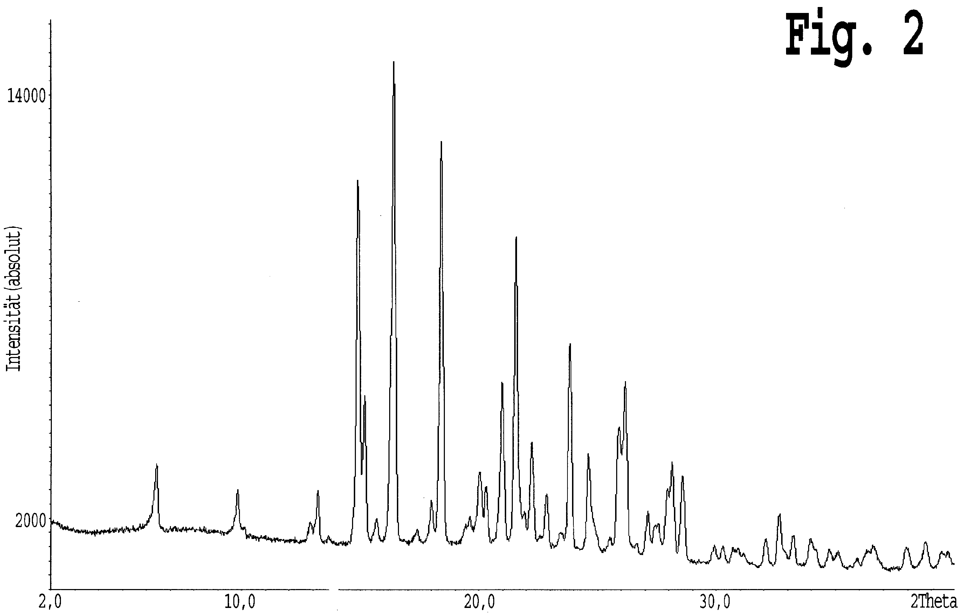

Ohne Stabilisierung zeigt Donepezil-Hydrochlorid der kristallinen polymorphen Form I die ausgeprägte Tendenz, sich verhältnismäßig schnell in die ebenfalls literaturbekannte kristalline polymorphe Form III umzuwandeln. Die kristalline polymorphe Form III von Donepezil-Hydrochlorid zeigt unter Verwendung des vorerwähnten Gerätes und bei den vorgenannten Geräte-/Messparametern das in der Fig. 2 gezeigte Pulver-Röntgenbeugungsmuster mit den signifikantesten Signalen bei den nachstehenden Beugungswinkeln 2θ mit der jeweilig zugeordneten relativen Intensität I/I₀

| Beugungswinkel 2θ | relative Intensität I/I₀ |
|---|---|
| [°] | [%] |
| 14,93 | 75,91 |
| 15,19 | 30,38 |
| 16,26 | 29,67 |
| 16,43 | 100 |
| 18,43 | 84,95 |
| 20,99 | 33,79 |
| 21,57 | 64,70 |
| 23,84 | 43,03 |
| 25,91 | 26,23 |
| 26,16 | 35,65 |

In der erfindungsgemäßen Zusammensetzung kommt die Stabilisierung der kristallinen Form I von Donepezil-Hydrochlorid vermutlich dadurch zu Stande, dass Donepezil-Hydrochlorid in direktem Kontakt mit Erdalkalimetallsalz der ortho-Phosphorsäure steht. Entsprechend ist es gemäß einer bevorzugten Ausführungsform der erfindungsgemäßen Zusammensetzung vorgesehen, dass das Donepezil-Hydrochlorid und das Erdalkalimetallsalz der Phosphorsäure in Form eines Gemisches in der Zusammensetzung vorliegen, vorzugsweise in homogen miteinander vermischter Form. Dadurch wird eine weitgehende Stabilisierung der polymorphen Form I von Donepezil-Hydrochlorid auf technisch einfache Weise gewährleistet.

Entsprechend einer bevorzugten Ausführungsform der erfindungsgemäßen Zusammensetzung ist es vorgesehen, dass das Donepezil-Hydrochlorid eine mittlere Partikelgröße in einem Bereich von 1 µm bis 50 µm aufweist.

Es konnte festgestellt werden, dass das Ausmaß der Stabilisierung des Donepezil-Hydrochlorids der polymorphen Form I durch das Erdalkalimetall-Phosphorsäuresalz von der mittleren Partikelgröße des Wirkstoffs abhängig ist. Und zwar ist die Stabilisierung besonders dann stark ausgeprägt, wenn die mittlere Partikelgröße in einem Bereich von 1 µm bis 50 µm liegt, bevorzugt in einem Bereich von 2 µm bis 35 µm und besonders bevorzugt in einem Bereich von 2 µm bis 25 µm.

Entsprechend ist es einer bevorzugten Ausführungsform der erfindungsgemäßen Zusammensetzung vorgesehen, dass das Donepezil-Hydrochlorid eine mittlere Partikelgröße in einem Bereich von 1 µm bis 50 µm aufweist, bevorzugt in einem Bereich von 2 µm bis 35 µm und besonders bevorzugt in einem Bereich von 2 µm bis 25 µm.

Unter "mittlere Partikelgröße" (D₅₀-Wert) wird im Rahmen der vorliegenden Erfindung die Partikelgröße verstanden, bei der 50 % der Partikel bezogen auf das Volumen kleiner als der D₅₀-Wert sind und 50 % der Partikel bezogen auf das Volumen größer sind als der D₅₀-Wert. Die Bestimmung der mittleren Partikelgröße erfolgt mittels Laserbeugung und Einsatz eines Gerätes mit der Bezeichnung "Mastersizer 2000" der Firma "Malvern Instruments". Die Bestimmung des D₅₀-Wertes wird erfindungsgemäß bevorzugt gemäß ISO/DIN 13320-1 durchgeführt, wobei die Messauswertung mittels der Mie-Theorie erfolgt, die im Stand der Technik bekannt ist. Alternativ dazu ist es erfindungsgemäß bevorzugt, dass die Messauswertung mittels der im Stand der Technik ebenfalls bekannten Fraunhofer-Methode erfolgt.

In der erfindungsgemäßen Zusammensetzung ist ein Erdalkalimetallsalz der ortho-Phosphorsäure (H₃PO₄) enthalten. Dabei kann im Prinzip jedes entsprechende Salz in der Zusammensetzung eingesetzt sein, das dem Fachmann für den erfindungsgemäßen Zweck als geeignet bekannt ist. Beispielsweise kann das Salz ein Erdalkalimetall-Dihydrogenphosphat mit dem Anion H₂PO₄⁻ sein (ein sogenanntes primäres Erdalkalimetallphosphat), ein Erdalkalimetall-Hydrogenphosphat mit dem Anion HPO₄²⁻ (ein sogenanntes sekundäres Erdalkalimetallphosphat) oder ein tertiäres Erdalkalimetallphosphat mit dem Anion PO₄³⁻. Erfindungsgemäß bevorzugt ist es jedoch, dass das Erdalkalimetallsalz der Phosphorsäure ein dibasisches sekundäres Erdalkalimetallphosphat mit dem Anion HPO₄²⁻ ist.

Bei dem Erdalkalimetallsalz der Phosphorsäure handelt es sich erfindungsgemäß bevorzugt um ein Magnesiumsalz oder um ein Calciumsalz, wobei insbesondere Calciumsalze bevorzugt sind. Beispiele für geeignete Calciumsalze sind Calciumphosphat (Ca₃(PO₄)₂), Calciumdihydrogenphosphat-Monohydrat (Ca(H₂PO₄)₂ × H₂O), Calciumhydrogenphosphat-Dihydrat (CaHPO₄ × 2H₂O) oder Calciumhydrogenphosphat-Dihydrat (CaHPO₄).

Gemäß einer weiter bevorzugten Ausführungsform der erfindungsgemäßen Zusammensetzung ist es vorgesehen, dass das Erdalkalimetallsalz der Phosphorsäure Calciumhydrogenphosphat ist, wobei das Calciumhydrogenphosphat in der Hydrat-Form Calciumhydrogenphosphat-Dihydrat (CaHPO₄ × 2H₂O) oder in der wasserfreien Form Calciumhydrogenphosphat-Anhydrat (CaHPO₄) vorliegen kann. Erfindungsgemäß bevorzugt ist das Calciumhydrogenphosphat-Dihydrat.

Entsprechend einer besonders bevorzugten Ausführungsform der erfindungsgemäßen Zusammensetzung ist es vorgesehen, dass das Calciumhydrogenphosphat ein Calciumhydrogenphosphat der Marke Emcompress^{®} (JRS Pharma GmbH & Co. KG, Rosenberg, Deutschland) ist. Unter der Marke Emcompress^{®} wird derzeit Calciumhydrogenphosphat in drei Qualitäten angeboten, nämlich Emcompress^{®} (CaHPO₄ × 2H₂O; monokline Kristallstruktur; Standardqualität), Emcompress^{®} Premium (CaHPO₄ × 2H₂O; monokline Kristallstruktur; erfüllt die Anforderungen des japanischen Arzneibuches (JP)) und Anhydrous Emcompress^{®} (wasserfreies CaHPO₄; trikline Kristallstruktur). Erfindungsgemäß bevorzugt sind alle drei der genannten Calciumhydrogenphosphat-Qualitäten der Marke Emcompress^{®}, wobei das Emcompress^{®} Premium erfindungsgemäß besonders bevorzugt ist.

Es konnte festgestellt werden, dass das Ausmaß der Stabilisierung des Donepezil-Hydrochlorids der polymorphen Form I durch das Erdalkalimetall-Phosphorsäuresalz auch von der mittleren Partikelgröße des Erdalkalimetallsalzes der Phosphorsäure abhängig ist. Und zwar ist die Stabilisierung besonders dann stark ausgeprägt, wenn die mittlere Partikelgröße in einem Bereich von 25 µm bis 710 µm liegt, bevorzugt in einem Bereich von 63 µm bis 500 µm und besonders bevorzugt in einem Bereich von 75 µm bis 425 µm. Entsprechend ist es gemäß einer bevorzugten Ausführungsform der erfindungsgemäßen Zusammensetzung vorgesehen, dass das Erdalkalimetallsalze der Phosphorsäure eine mittlere Partikelgröße in einem Bereich von 25 µm bis 710 µm aufweist, bevorzugt in einem Bereich von 63 µm bis 500 µm und besonders bevorzugt in einem Bereich von 75 µm bis 425 µm.

In der erfindungsgemäßen Zusammensetzung ist Donepezil-Hydrochlorid der polymorphen Form I sowie ein Erdalkalimetallsalz der Phosphorsäure enthalten, welche die kristalline polymorphe Form I stabilisiert. Es konnte festgestellt werden, dass das Ausmaß der Stabilisierung auch abhängig ist von dem Erdalkalimetallphosphat/Donepezil-Hydrochlorid-Molverhältnis der Zusammensetzung. Und zwar ist bei einem Molverhätnis kleiner als 0,01 die stabilisierende Wirkung nicht besonders stark ausgeprägt, während bei einem Molverhältnis oberhalb von 50 in der Regel kaum noch Verbesserungen hinsichtlich der Stabilität erzielt werden können. Entsprechend ist es gemäß einer weiter bevorzugten Ausführungsform der erfindungsgemäßen Zusammensetzung vorgesehen, dass das Molverhältnis von dem Erdalkalimetallsalz der Phosphorsäure zu dem Donepezil-Hydrochlorid in einem Bereich von 0,01 bis 50 liegt, vorzugsweise in einem Bereich von 0,1 bis 40 und besonders bevorzugt in einem Bereich von 5 bis 20.

Darüber hinaus konnte festgestellt werden, dass das Ausmaß der Stabilisierung der polymorphen Form I des Donepezil-Hydrochlorids durch das Erdalkalimetallphosphat nicht nur vom Molverhältnis der genannten Substanzen, sondern auch vom Anteil der erfindungsgemäßen Zusammensetzung an Donepezil-Hydrochlorid abhängt. Erfindungsgemäß bevorzugt ist es, dass der Anteil der Zusammensetzung an Donepezil-Hydrochlorid (Trockenmasse) 1 Gew.-% bis 10 Gew.-% beträgt bezogen auf Gesamtgewicht der Zusammensetzung, bevorzugt 1,5 Gew.-% bis 8 Gew.-% und besonders bevorzugt 2 Gew.-% bis 5 Gew.-%.

Entsprechend einer weiter bevorzugten Ausführungsform der erfindungsgemäßen Zusammensetzung ist es vorgesehen, dass die Zusammensetzung eine Einzeldosisform ist. Eine erfindungsgemäß bevorzugte Einzeldosisform ist eine Tablette, zum Beispiel eine Filmtablette, eine Kapsel oder ein Pellet.

Für den Fall, dass die erfindungsgemäße feste pharmazeutische Zusammensetzung eine Einzeldosisform ist wie etwa eine Tablette oder eine Kapsel, enthält diese erfindungsgemäß bevorzugt 2 mg bis 20 mg Donepezil-Hydrochlorid (Trockenmasse) der polymorphen Form I, mehr bevorzugt 4 mg bis 12 mg und weiter bevorzugt 5 mg oder 10 mg Donepezil-Hydrochlorid der polymorphen Form I.

Gemäß einer weiter bevorzugten Ausführungsform der erfindungsgemäßen Zusammensetzung ist es vorgesehen, dass die Zusammensetzung direktverpresst ist. Unter "direktverpresst" wird dabei in Analogie zur Direkttablettierung verstanden, dass die feste Zusammensetzung durch Verpressen eines entsprechenden Pulvergemisches ohne weitere Vorbehandlung hergestellt ist.

Entsprechend einer weiteren bevorzugten Ausführungsform der Erfindung umfasst die Zusammensetzung ferner einen oder mehrere pharmazeutische Hilfsstoffe. Erfindungsgemäß bevorzugte Hilfsstoffe sind ausgewählt aus der Gruppe bestehend aus einem Füllmittel, Füllmittelgemisch, Sprengmittel, Sprengmittelgemisch, Bindemittel,

Bindemittelgemisch, Fließregulierungsmittel, Fließregulierungsmittelgemisch, Schmiermittel, Schmiermittelgemisch, Formentrennmittel und einem Formentrennmittelgemisch.

Der Anteil der erfindungsgemäßen pharmazeutischen Zusammensetzung an pharmazeutischem/n Hilfsstoff/en beträgt erfindungsgemäß bevorzugt 10 Gew.-% bis 90 Gew.-%, weiter bevorzugt 70 Gew.-% bis 82 Gew.-% bezogen auf das Gesamtgewicht der Zusammensetzung.

Die vorliegende Erfindung betrifft ferner ein erstes Verfahren zur Herstellung einer erfindungsgemäßen Zusammensetzung, umfassend die Schritte
a) Vermischen von pulverförmigem kristallinem Donepezil-Hydrochlorid der polymorphen Form I mit einem unter Verwendung von Cu-K_{α1}-Strahlung erhaltenen Pulver-Röntgenbeugungsmuster, das die zehn intensitätsstärksten Signale bei den nachstehenden Beugungswinkeln 2θ mit der jeweilig zugeordneten relativen Intensität I/I₀ aufweist

| Beugungswinkel 2θ | relative Intensität I/I₀ |
|---|---|
| [°] | [%] |
| 4,91 | 48,43 |
| 12,64 | 100 |
| 13,07 | 58,57 |
| 13,84 | 22,05 |
| 16,07 | 35,24 |
| 16,87 | 26,21 |
| 21,16 | 71,72 |
| 23,00 | 24,30 |
| 23,90 | 33,38 |
| 24,17 | 20,49 |

mit einem pulverförmigen Erdalkalimetallsalz der ortho-Phosphorsäure, wobei ein pulverförmiges Donepezil-Hydrochlorid/Erdalkalimetall-Phosphorsäuresalz-Gemisch erhalten wird;
b) Verpressen des pulverförmigen Donepezil-Hydrochlorid/Erdalkalimetall-Phosphorsäuresalz-Gemisches unter Erhalt eines Presslings, vorzugsweise einer Tablette oder eines mittels Trockengranulierung zu einem Granulat verarbeitbaren Komprimats, beispielsweise ein Brikett.

Die vorliegende Erfindung betrifft ferner ein zweites Verfahren zur Herstellung einer erfindungsgemäßen Zusammensetzung, umfassend die Schritte
a) Vermischen von pulverförmigem kristallinem Donepezil-Hydrochlorid der polymorphen Form I mit einem unter Verwendung von Cu-K_{α1}-Strahlung erhaltenen Pulver-Röntgenbeugungsmuster, das die zehn intensitätsstärksten Signale bei den nachstehenden Beugungswinkeln 2θ mit der jeweilig zugeordneten relativen Intensität I/I₀ aufweist

| Beugungswinkel 2θ | relative Intensität I/I₀ |
|---|---|
| [°] | [%] |
| 4,91 | 48,43 |
| 12,64 | 100 |
| 13,07 | 58,57 |
| 13,84 | 22,05 |
| 16,07 | 35,24 |
| 16,87 | 26,21 |
| 21,16 | 71,72 |
| 23,00 | 24,30 |
| 23,90 | 33,38 |
| 24,17 | 20,49 |

mit einem pulverförmigen Erdalkalimetallsalz der ortho-Phosphorsäure, wobei ein pulverförmiges Donepezil-Hydrochlorid/Erdalkalimetall-Phosphorsäuresalz-Gemisch erhalten wird;
b) Granulieren des pulverförmigen Donepezil-Hydrochlorid/Erdalkalimetall-Phosphorsäuresalz-Gemisches, vorzugsweise mittels Feuchtgranulierung oder Trockengranulierung.

Die vorliegende Erfindung betrifft ferner eine pulverförmige Vormischung (Premix), insbesondere zur Herstellung einer erfindungsgemäßen Zusammensetzung, umfassend
a) kristallines Donepezil-Hydrochlorid der polymorphen Form I mit einem unter Verwendung von Cu-K_{α1}-Strahlung erhaltenen Pulver-Röntgenbeugungsmuster, das die zehn intensitätsstärksten Signale bei den nachstehenden Beugungswinkeln 2θ mit der jeweilig zugeordneten relativen Intensität I/I₀ aufweist

| Beugungswinkel 2θ | relative Intensität I/I₀ |
|---|---|
| [°] | [%] |
| 4,91 | 48,43 |
| 12,64 | 100 |
| 13,07 | 58,57 |
| 13,84 | 22,05 |
| 16,07 | 35,24 |
| 16,87 | 26,21 |
| 21, 16 | 71,72 |
| 23,00 | 24,30 |
| 23,90 | 33,38 |
| 24,17 | 20,49 |

b) ein Erdalkalimetallsalz der ortho-Phosphorsäure.

In der erfindungsgemäßen pulvrigen Vormischung ist das Donepezil-Hydrochlorid der polymorphen Form I weitgehend stabil. Die Vormischung ist daher zur Lagerung und zum Transport von Donepezil-Hydrochlorid der polymorphen Form I geeignet.

Entsprechend einer bevorzugten Ausführungsform der erfindungsgemäßen Vormischung weist diese eine Zusammensetzung wie die erfindungsgemäße feste pharmazeutische Zusammensetzung auf. Dadurch ist die Möglichkeit gegeben, dass die Vormischung direkt zur erfindungsgemäßen Zusammensetzung verarbeitet werden kann.

Entsprechend einer bevorzugten Ausführungsform der erfindungsgemäßen Vormischung ist diese derart zusammengesetzt, dass diese zur Direkttablettierung geeignet ist.

Die vorliegende Erfindung betrifft ferner die Verwendung einer erfindungsgemäßen Zusammensetzung oder einer erfindungsgemäßen Vormischung zur Herstellung eines Medikamentes zur Behandlung von seniler Demenz, insbesondere zur Behandlung von seniler Demenz vom Alzheimer-Typ.

Die nachfolgenden Ausführungs- und Vergleichsbeispiele sowie die Versuche zur Lagerungsstabilität dienen im Zusammenhang mit der Zeichnung der Erläuterung der Erfindung. Es zeigen:
- Fig. 1:: PXRD-Spektrum von Donepezil-Hydrochlorid der polymorphen Form I;
- Fig. 2:: PXRD-Spektrum von Donepezil-Hydrochlorid der polymorphen Form III;
- Fig. 3:: PXRD-Spektrum einer nicht gelagerten Filmtablette gemäß Ausführungsbeispiel 1;
- Fig. 4:: PXRD-Spektrum (untere Kurve) von einer Filmtablette gemäß Ausführungsbeispiel 1 nach fünfmonatiger Lagerung in einem PVCD-Blister bei 40 °C und 75 % r.h. und PXRD-Spektrum (obere Kurve) von einer analogen Placebo-Filmtablette;
- Fig. 5:: PXRD-Spektrum einer nicht gelagerten Filmtablette gemäß Vergleichsbeispiel 1;
- Fig. 6:: PXRD-Spektrum (untere Kurve) von einer Filmtablette gemäß Vergleichsbeispiel 1 nach dreimonatiger Lagerung in einem PVCD-Blister bei 40 °C und 75 % r.h. und PXRD-Spektrum (obere Kurve) von einer analogen-Placebo-Filmtablette.

### Ausführungsbeispiel 1:

Es wurden Filmtabletten als erfindungsgemäße Zusammensetzung einer ersten Ausführungsform enthaltend 5,0 mg Donepezil-Hydrochlorid der polymorphen Form I hergestellt. Die Tabletten wiesen die in der Tabelle 1 angegebene Zusammensetzung auf:

**Tabelle 1:**

| **Substanz** | **Menge pro Tablette [mg]** | **Funktion der Substanz** |
|---|---|---|
| **Tablettenkern** | | |
| Donepezil-Hydrochlorid (Form I) | 5,0 | Wirkstoff |
| wasserfreie Lactose | 87,0 | Füllmittel |
| vorgekleisterte Stärke | 21,0 | Sprengmittel |
| Calciumhydrogenphosphat-Dihydrat | 21,0 | Füllmittel, Bindemittel |
| Talkum | 3,0 | Fließregulierungsmittel |
| Magnesiumstearat | 1,5 | Schmiermittel |
| hochdisperses Siliciumdioxid | 0,5 | Fließregulierungsmittel |

| **Film** | | |
|---|---|---|
| Hypromellose | 1,5 | Filmbildner |
| Hydroxypropylcellulose | 1,5 | Filmbildner |
| Talkum | 0,5 | Schmiermittel |
| Titandioxid | 1,5 | Pigment |
| | | |
| Gesamtgewicht der Filmtablette | 144,0 | |

Die Herstellung der Filmtabletten erfolgte wie nachstehend beschrieben.

Donepezil-Hydrochlorid der polymorphen Form I, Calciumhydrogenphosphat-Dihydrat der Marke Emcompress^{®} (JRS Pharma GmbH & Co. KG, Rosenberg, Deutschland), wasserfreie Lactose, vorgekleisterte Stärke, Talkum und hochdisperses Siliciumdioxid wurden gesiebt, abgewogen und in einem Mischer bis zur Homogenität miteinander vermischt. Zu dieser Mischung wurde Magnesiumstearat gegeben und untergemischt. Das so erhaltene Gemisch wurde mittels einer konventionellen Tablettenpresse zu Tablettenkernen direktverpresst.

Die so erhaltenen Tablettenkerne wurden in einer konventionellen Filmbeschichtungsvorrichtung durch Besprühen mit einer Beschichtungssuspension befilmt. Die Beschichtungssuspension wurde hergestellt durch Vereinigung einer wässrigen Lösung von Hypromellose und Hydroxypropylcellulose mit einer Suspension von Talkum und Titandioxid in Wasser.

Zu den in Rede stehenden Filmtabletten der ersten Ausführungsform der erfindungsgemäßen Zusammensetzung wurden analoge Placebo-Filmtabletten hergestellt, d.h. Filmtabletten gleicher Zusammensetzung jedoch ohne den Wirkstoff Donepezil-Hydrochlorid.

### Ausführungsbeispiel 2:

Es wurden Filmtabletten als erfindungsgemäße Zusammensetzung einer zweiten Ausführungsform enthaltend 10,0 mg Donepezil-Hydrochlorid der polymorphen Form I hergestellt. Die Tabletten wiesen die in der Tabelle 2 angegebene Zusammensetzung auf:

**Tabelle 2:**

| **Substanz** | **Menge pro Tablette [mg]** | **Funktion der Substanz** |
|---|---|---|
| **Tablettenkern** | | |
| Donepezil-Hydrochlorid (Form I) | 10,0 | Wirkstoff |
| wasserfreie Lactose | 174,0 | Füllmittel |
| vorgekleisterte Stärke | 42,0 | Sprengmittel |
| Calciumhydrogenphosphat-Dihydrat | 42,0 | Füllmittel, Bindemittel |
| Talkum | 6,0 | Fließregulierungsmittel |
| Magnesiumstearat | 3,0 | Schmiermittel |
| hochdisperses Siliciumdioxid | 1,5 | Fließregulierungsmittel |

| **Film** | | |
|---|---|---|
| Hypromellose | 3,0 | Filmbildner |
| Hydroxypropylcellulose | 3,0 | Filmbildner |
| Talkum | 1,0 | Schmiermittel |
| Titandioxid | 3,0 | Pigment |
| Eisenoxid (gelb; E 172) | 0,1 | Pigment |
| Gesamtgewicht der Filmtablette | 288,6 | |

Die Herstellung der Filmtabletten erfolgte analog dem Ausführungsbeispiel 1, wobei abweichend davon die Suspension von Talkum und Titandioxid in Wasser ferner das Eisenoxid (gelb) enthielt.

### Vergleichsbeispiel 1:

Es wurden Filmtabletten enthaltend 5,0 mg Donepezil-Hydrochlorid der polymorphen Form I hergestellt. Die Tabletten waren frei von einem Erdalkalimetallsalz der ortho-Phosphorsäure und wiesen die in der Tabelle 3 angegebene Zusammensetzung auf:

**Tabelle 3:**

| **Substanz** | **Menge pro Tablette [mg]** | **Funktion der Substanz** |
|---|---|---|
| **Tablettenkern** | | |
| Donepezil-Hydrochlorid (Form I) | 5,0 | Wirkstoff |
| wasserfreie Lactose | 108,0 | Füllmittel |
| vorgekleisterte Stärke | 21,0 | Sprengmittel |
| Talkum | 3,0 | Fließregulierungsmittel |
| Magnesiumstearat | 1,5 | Schmiermittel |
| hochdisperses Siliciumdioxid | 0,5 | Fließregulierungsmittel |

| **Film** | | |
|---|---|---|
| Hypromellose | 1,5 | Filmbildner |
| Hydroxypropylcellulose | 1,5 | Filmbildner |
| Talkum | 0,5 | Schmiermittel |
| Titandioxid | 1,5 | Pigment |
| | | |
| Gesamtgewicht der Filmtablette | 144,0 | |

Die Herstellung der Filmtabletten erfolgte analog dem Ausführungsbeispiel 1.

Zu den in Rede stehenden Filmtabletten des Vergleichsbeispiels 1 wurden analoge Placebo-Filmtabletten hergestellt, d.h. Filmtabletten gleicher Zusammensetzung jedoch ohne den Wirkstoff Donepezil-Hydrochlorid.

### Vergleichsbeispiel 2:

Es wurden Filmtabletten als Vergleichsbeispiel enthaltend 10,0 mg Donepezil-Hydrochlorid der polymorphen Form I hergestellt. Die Tabletten waren frei von einem Erdalkalimetallsalz der ortho-Phosphorsäure und wiesen die in der Tabelle 4 angegebene Zusammensetzung auf:

**Tabelle 4:**

| **Substanz** | **Menge pro Tablette [mg]** | **Funktion der Substanz** |
|---|---|---|
| **Tablettenkern** | | |
| Donepezil-Hydrochlorid (Form I) | 10,0 | Wirkstoff |
| wasserfreie Lactose | 216,0 | Füllmittel |
| vorgekleisterte Stärke | 42,0 | Sprengmittel |
| Talkum | 6,0 | Fließregulierungsmittel |
| Magnesiumstearat | 3,0 | Schmiermittel |
| hochdisperses Siliciumdioxid | 1,5 | Fließregulierungsmittel |

| **Film** | | |
|---|---|---|
| Hypromellose | 3,0 | Filmbildner |
| Hydroxypropylcellulose | 3,0 | Filmbildner |
| Talkum | 1,0 | Schmiermittel |
| Titandioxid | 3,0 | Pigment |
| Eisenoxid (gelb; E 172) | 0,1 | Pigment |
| Gesamtgewicht der Filmtablette | 288,6 | |

Die Herstellung der Filmtabletten erfolgte analog dem Ausführungsbeispiel 2.

### Versuche zur Lagerungsstabilität:

Zur Bestimmung der Stabilität der polymorphen Form I des Wirkstoffs Donepezil-Hydrochlorid in den Filmtabletten gemäß Ausführungsbeispiel 1 und Vergleichsbeispiel 1 wurden die jeweiligen Filmtabletten vor und nach Lagerung sowie die jeweiligen Placebo-Filmtabletten mittels Pulver-Röntgendiffraktometrie untersucht, wobei sogenannte Pulver-Röntgendiffraktions(PXRD)-Spektren erhalten wurden.

Für die PXRD-Messungen wurden die Tablettenkerne der Filmtabletten/Placebo-Filmtabletten durch Abkratzen der Befilmung freigelegt und zu einem Pulver vermahlen.

Für die Pulver-Röntgendiffraktions-Messungen wurde das wie vorstehend beschrieben erhaltene Pulver bzw. der reine pulvrige Wirkstoff der polymorphen Form I bzw. III zwischen zwei Folien der Marke Mylar^{®} gehalten. Die PXRD-Messungen wurden mittels eines Transmissionsdiffraktometer-Gerätes der Bezeichnung STADI P der Firma Stoe & Cie GmbH, Darmstadt, Deutschland, durchgeführt. Die Geräte-/Messparameter waren wie folgt:
- Wellenlänge: λ=0,154056 nm (Cu-K_{α1}-Strahlung)
- Monochromator: gebogen, Ge(111)
- Spannung: U=40 kV
- Stromstärke: I=30 mA
- Winkelbereich: 2θ=2° bis 40°
- Schrittweite: Δ 2θ=0,2°
- Messzeit: 20 s pro 0,2°-Schritt
- Detektor: linearer ortsempfindlicher Detektor (linear position sensitive detector (PSD))
- Probenträgermaske: Durchmesser: 8 mm

Die qualitative Phasenanalyse der Spektren erfolgte anhand erstellter Signallisten für die entsprechenden Formen.

Die Lagerung der Filmtabletten gemäß Ausführungsbeispiel 1 und Vergleichsbeispiel 1 erfolgte in einem Polyvinylidenchlorid (PVDC)-Blister (60 g/m²) für einen Zeitraum von fünf bzw. drei Monaten bei einer Temperatur von 40 °C und einer relativen Luftfeuchtigkeit (r.h.) von 75 %.

In der Fig. 1 und 2 ist ein PXRD-Spektrum von reinem Donepezil-Hydrochlorid der polymorphen Form I bzw. III gezeigt.

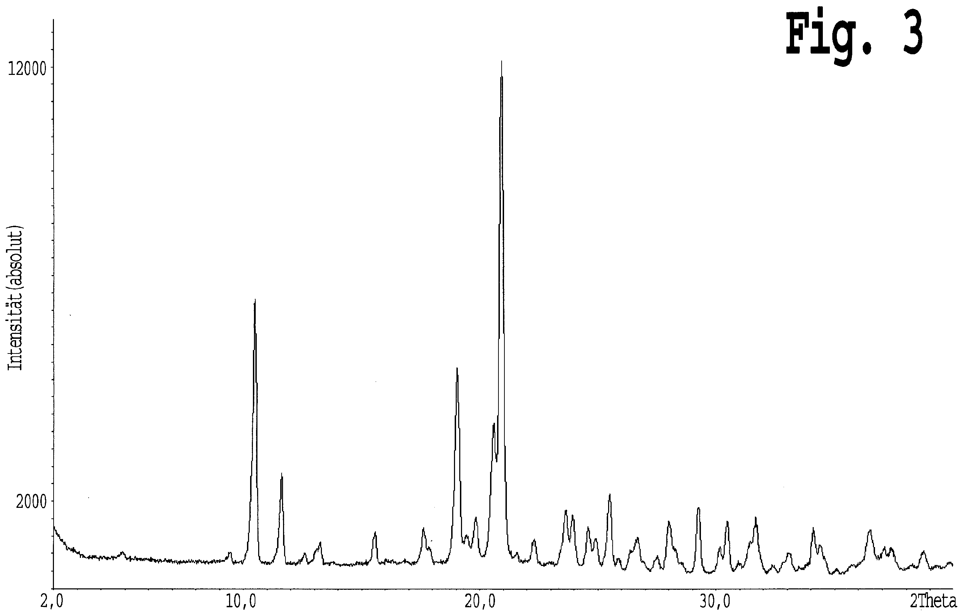

Fig. 3 zeigt ein PXRD-Spektrum nicht gelagerter Filmtabletten gemäß dem Ausführungsbeispiel 1. Wie deutlich zu erkennen ist, enthalten die Filmtabletten gemäß Ausführungsbeispiel 1 direkt nach ihrer Herstellung ausschließlich Donepezil-Hydrochlorid der polymorphen Form I; es ist das für die Form I charakteristische Signal bei 2Theta = 4,91° zu erkennen, jedoch nicht das für die Form III charakteristische Signal bei 2Theta = 14,93°.

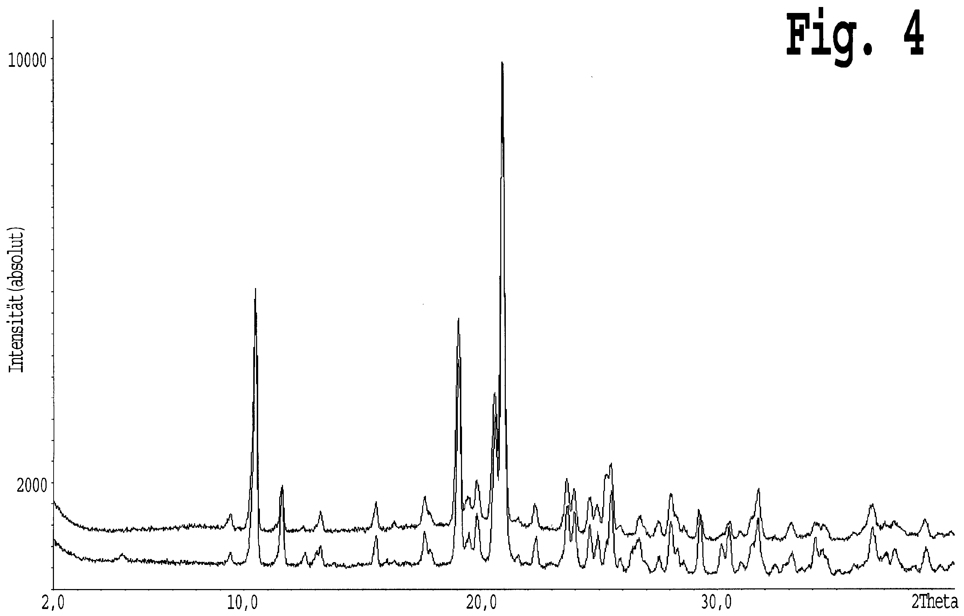

In der Fig. 4 ist ein PXRD-Spektrum (untere Kurve) von einer Filmtablette gemäß Ausführungsbeispiel 1 nach fünfmonatiger Lagerung in einem PVCD-Blister bei 40 °C und 75 % r.h. gezeigt sowie ein PXRD-Spektrum (obere Kurve) von einer analogen Placebo-Filmtablette. Wie mit Verweis auf das Vorhandensein und die Intensität des Signals bei 2Theta = 4,91° in der unteren Kurve deutlich zu erkennen ist, enthalten die Filmtabletten des Ausführungsbeispiels 1 auch nach der Lagerung ausschließlich Donepezil-Hydrochlorid der polymorphen Form I. In der erfindungsgemäßen Zusammensetzung ist Donepezil-Hydrochlorid der polymorphen Form I demgemäß selbst nach Lagerung unter extremen Stressbedingungen umwandlungsstabil.

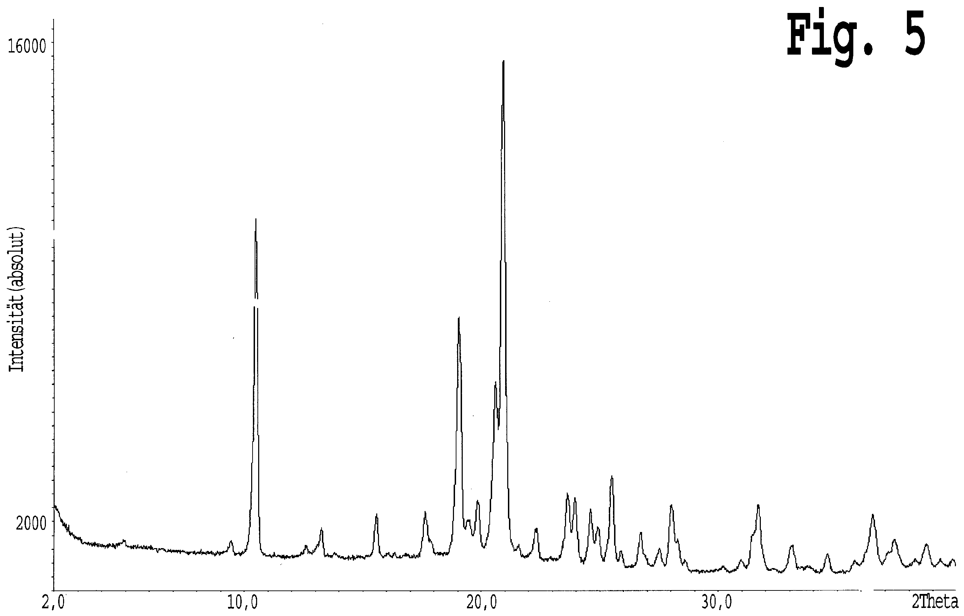

Fig. 5 zeigt ein PXRD-Spektrum nicht gelagerter Filmtabletten gemäß dem Vergleichsbeispiel 1. Wie deutlich zu erkennen ist, enthalten diese Filmtabletten direkt nach ihrer Herstellung ausschließlich Donepezil-Hydrochlorid der polymorphen Form I; es ist das für die Form I charakteristische Signal bei 2Theta = 4,91° zu erkennen, jedoch nicht das für die Form III charakteristische Signal bei 2Theta = 14,93°.

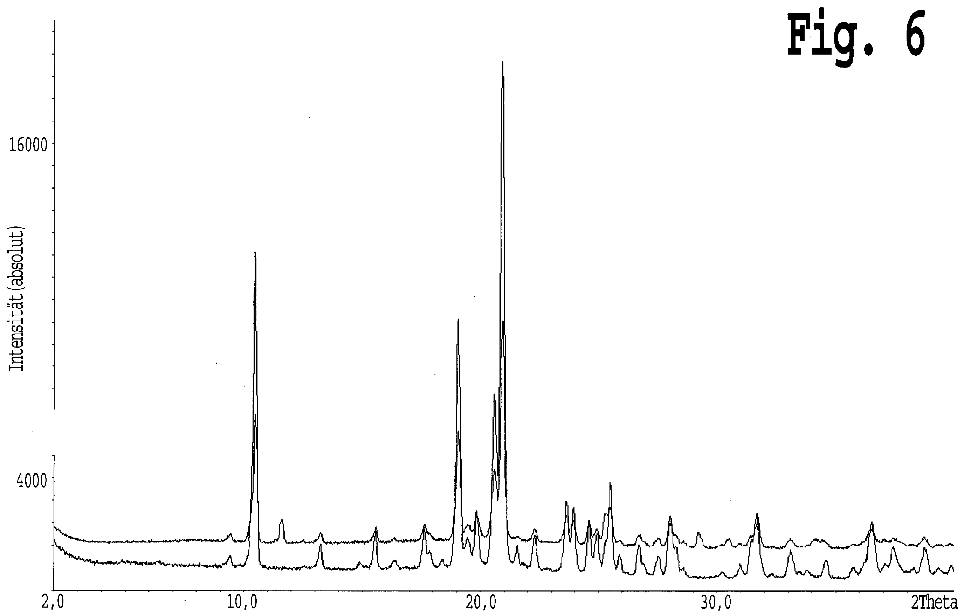

In der Fig. 6 ist ein PXRD-Spektrum (untere Kurve) von einer Filmtablette gemäß Vergleichsbeispiel 1 nach dreimonatiger Lagerung in einem PVCD-Blister bei 40 °C und 75 % r.h. gezeigt sowie ein PXRD-Spektrum (obere Kurve) von einer analogen Placebo-Filmtablette. Wie mit Verweis auf das Nichtvorhandensein eines Signals bei 2Theta = 4,91° (charakteristisch für Form I) in der unteren Kurve und des Vorhandenseins eines Signals bei 2Theta = 14,93° (charakteristisch für Form III) deutlich zu erkennen ist, ist in der Zusammensetzung des Vergleichsbeispiels 1, die frei von einem Erdalkalimetallsalz der ortho-Phosphorsäure ist, Donepezil-Hydrochlorid der polymorphen Form I nicht umwandlungsstabil, sondern lagert sich im Laufe der Zeit nahezu ausschließlich in die polymorphe Form III um.

## Patentansprüche

1. Feste pharmazeutische Zusammensetzung, umfassend
a) kristallines Donepezil-Hydrochlorid der polymorphen Form I mit einem unter Verwendung von Cu-K_{α1}-Strahlung erhaltenen Pulver-Röntgenbeugungsmuster, das die zehn intensitätsstärksten Signale bei den nachstehenden Beugungswinkeln 2θ mit der jeweilig zugeordneten relativen Intensität I/I₀ aufweist
| Beugungswinkel 2θ | relative Intensität I/I₀ |
|---|---|
| [°] | [%] |
| 4,91 | 48,43 |
| 12,64 | 100 |
| 13,07 | 58,57 |
| 13,84 | 22,05 |
| 16,07 | 35,24 |
| 16,87 | 26,21 |
| 21,16 | 71,72 |
| 23,00 | 24,30 |
| 23,90 | 33,38 |
| 24,17 | 20,49 |
und
b) ein Erdalkalimetallsalz der ortho-Phosphorsäure.

2. Zusammensetzung nach Anspruch 1, **dadurch gekennzeichnet, dass** das Donepezil-Hydrochlorid eine mittlere Partikelgröße in einem Bereich von 1 µm bis 50 µm aufweist.

3. Zusammensetzung nach einem der voranstehenden Ansprüche, **dadurch gekennzeichnet, dass** das Erdalkalimetallsalz der Phosphorsäure Calciumhydrogenphosphat ist.

4. Zusammensetzung nach einem der voranstehenden Ansprüche, **dadurch gekennzeichnet, dass** das Molverhältnis von dem Erdalkalimetallsalz der Phosphorsäure zu dem Donepezil-Hydrochlorid in einem Bereich von 0,01 bis 50 liegt.

5. Zusammensetzung nach einem der voranstehenden Ansprüche, **dadurch gekennzeichnet, dass** der Anteil der Zusammensetzung an dem Donepezil-Hydrochlorid 1 Gew.-% bis 10 Gew.-% beträgt bezogen auf Gesamtgewicht der Zusammensetzung.

6. Zusammensetzung nach einem der voranstehenden Ansprüche, **dadurch gekennzeichnet, dass** die Zusammensetzung direktverpresst ist.

7. Verfahren zur Herstellung einer Zusammensetzung nach einem der Ansprüche 1 bis 6, umfassend die Schritte
a) Vermischen von pulverförmigem kristallinem Donepezil-Hydrochlorid der polymorphen Form I mit einem unter Verwendung von Cu-K_{α1}-Strahlung erhaltenen Pulver-Röntgenbeugungsmuster, das die zehn intensitätsstärksten Signale bei den nachstehenden Beugungswinkeln 2θ mit der jeweilig zugeordneten relativen Intensität I/I₀ aufweist
| Beugungswinkel 2θ | relative Intensität I/I₀ |
|---|---|
| [°] | [%] |
| 4,91 | 48,43 |
| 12,64 | 100 |
| 13,07 | 58,57 |
| 13,84 | 22,05 |
| 16,07 | 35,24 |
| 16,87 | 26,21 |
| 21,16 | 71,72 |
| 23,00 | 24,30 |
| 23,90 | 33,38 |
| 24,17 | 20,49 |
mit einem pulverförmigen Erdalkalimetallsalz der ortho-Phosphorsäure, wobei ein pulverförmiges Donepezil-Hydrochlorid/Erdalkalimetall-Phosphorsäuresalz-Gemisch erhalten wird;
und
b) Verpressen des pulverförmigen Donepezil-Hydrochlorid/Erdalkalimetall-Phosphorsäuresalz-Gemisches unter Erhalt eines Presslings.

8. Verfahren zur Herstellung einer Zusammensetzung nach einem der Ansprüche 1 bis 6, umfassend die Schritte
a) Vermischen von pulverförmigem kristallinem Donepezil-Hydrochlorid der polymorphen Form I mit einem unter Verwendung von Cu-K_{α1}-Strahlung erhaltenen Pulver-Röntgenbeugungsmuster, das die zehn intensitätsstärksten Signale bei den nachstehenden Beugungswinkeln 2θ mit der jeweilig zugeordneten relativen Intensität I/I₀ aufweist
| Beugungswinkel 2θ | relative Intensität I/I₀ |
|---|---|
| [°] | [%] |
| 4,91 | 48,43 |
| 12,64 | 100 |
| 13,07 | 58,57 |
| 13,84 | 22,05 |
| 16,07 | 35,24 |
| 16,87 | 26,21 |
| 21,16 | 71,72 |
| 23,00 | 24,30 |
| 23,90 | 33,38 |
| 24,17 | 20,49 |
mit einem pulverförmigen Erdalkalimetallsalz der ortho-Phosphorsäure, wobei ein pulverförmiges Donepezil-Hydrochlorid/Erdalkalimetall-Phosphorsäuresalz-Gemisch erhalten wird;
und
b) Granulieren des pulverförmigen Donepezil-Hydrochlorid/Erdalkalimetall-Phosphorsäuresalz-Gemisches.

9. Pulverförmige Vormischung, umfassend
a) kristallines Donepezil-Hydrochlorid der polymorphen Form I mit einem unter Verwendung von Cu-K_{α1}-Strahlung erhaltenen Pulver-Röntgenbeugungsmuster, das die zehn intensitätsstärksten Signale bei den nachstehenden Beugungswinkeln 2θ mit der jeweilig zugeordneten relativen Intensität I/I₀ aufweist
| Beugungswinkel 2θ | relative Intensität I/I₀ |
|---|---|
| [°] | [%] |
| 4,91 | 48,43 |
| 12,64 | 100 |
| 13,07 | 58,57 |
| 13,84 | 22,05 |
| 16,07 | 35,24 |
| 16,87 | 26,21 |
| 21,16 | 71,72 |
| 23,00 | 24,30 |
| 23,90 | 33,38 |
| 24,17 | 20,49 |
und
b) ein Erdalkalimetallsalz der ortho-Phosphorsäure.

10. Verwendung einer Zusammensetzung nach einem der Ansprüche 1 bis 6 oder einer Vormischung nach Anspruch 9 zur Herstellung eines Medikamentes zur Behandlung von seniler Demenz.

## Claims

1. Solid pharmaceutical composition comprising
a) crystalline donepezil hydrochloride in polymorphic form I, having a powder X-ray diffraction pattern, obtained using Cu-k_{α1} radiation, in which the ten signals of greatest intensity are at the following diffraction angles 2θ with the respectively assigned relative intensity I/I₀
| diffraction angle 2θ | relative intensity I/I₀ |
|---|---|
| [°] | [%] |
| 4.91 | 48.43 |
| 12.64 | 100 |
| 13.07 | 58.57 |
| 13.84 | 22.05 |
| 16.07 | 35.24 |
| 16.87 | 26.21 |
| 21.16 | 71.72 |
| 23.00 | 24.30 |
| 23.90 | 33.38 |
| 24.17 | 20.49 |
and
b) an alkaline earth metal salt of orthophosphoric acid.

2. Composition according to Claim 1, **characterized in that** the donepezil hydrochloride has an average particle size in a range from 1 µm to 50 µm.

3. Composition according to either of the preceding claims, **characterized in that** the alkaline earth metal salt of phosphoric acid is calcium hydrogenphosphate.

4. Composition according to any of the preceding claims, **characterized in that** the molar ratio of the alkaline earth metal salt of phosphoric acid to the donepezil hydrochloride is in a range from 0.01 to 50.

5. Composition according to any of the preceding claims, **characterized in that** the proportion of the donepezil hydrochloride in the composition is 1 wt% to 10 wt% based on total composition weight.

6. Composition according to any of the preceding claims, **characterized in that** the composition has undergone direct compression.

7. Method for producing a composition according to any of Claims 1 to 6, comprising the steps of
a) mixing pulverulent crystalline donepezil hydrochloride in polymorphic form I, having a powder X-ray diffraction pattern, obtained using Cu-K_{α1} radiation, in which the ten signals of greatest intensity are at the following diffraction angles 2θ with the respectively assigned relative intensity I/I₀
| diffraction angle 2θ | relative intensity I/I₀ |
|---|---|
| [°] | [%] |
| 4.91 | 48.43 |
| 12.64 | 100 |
| 13.07 | 58.57 |
| 13.84 | 22.05 |
| 16.07 | 35.24 |
| 16.87 | 26.21 |
| 21.16 | 71.72 |
| 23.00 | 24.30 |
| 23.90 | 33.38 |
| 24.17 | 20.49 |
with a pulverulent alkaline earth metal salt of orthophosphoric acid, to give a pulverulent donepezil hydrochloride/phosphoric acid alkaline earth metal salt mixture;
and
b) compressing the pulverulent donepezil hydrochloride/phosphoric acid alkaline earth metal salt mixture to give a compact.

8. Method for producing a composition according to any of Claims 1 to 6, comprising the steps of
a) mixing pulverulent crystalline donepezil hydrochloride in polymorphic form I, having a powder X-ray diffraction pattern, obtained using Cu-K_{α1} radiation, in which the ten signals of greatest intensity are at the following diffraction angles 2θ with the respectively assigned relative intensity I/I₀
| diffraction angle 2θ | relative intensity I/I₀ |
|---|---|
| [°] | [%] |
| | 48.43 |
| 12.64 | 100 |
| 13.07 | 58.57 |
| 13.84 | 22.05 |
| 16.07 | 35.24 |
| 16.87 | 26.21 |
| 21.16 | 71.72 |
| 23.00 | 24.30 |
| 23.90 | 33.38 |
| 24.17 | 20.49 |
with a pulverulent alkaline earth metal salt of orthophosphoric acid, to give a pulverulent donepezil hydrochloride/phosphoric acid alkaline earth metal salt mixture;
and
b) granulating the pulverulent donepezil hydrochloride/phosphoric acid alkaline earth metal salt mixture.

9. Pulverulent premix comprising
a) crystalline donepezil hydrochloride in polymorphic form I, having a powder X-ray diffraction pattern, obtained using Cu-K_{α1} radiation, in which the ten signals of greatest intensity are at the following diffraction angles 2θ with the respectively assigned relative intensity I/I₀
| diffraction angle 2θ | relative intensity I/I₀ |
|---|---|
| [°] | [%] |
| 4.91 | 48.43 |
| 12.64 | 100 |
| 13.07 | 58.57 |
| 13.84 | 22.05 |
| 16.07 | 35.24 |
| 16.87 | 26.21 |
| 21.16 | 71.72 |
| 23.00 | 24.30 |
| 23.90 | 33.38 |
| 24.17 | 20.49 |
and
b) an alkaline earth metal salt of orthophosphoric acid.

10. Use of a composition according to any of Claims 1 to 6 or of a premix according to Claim 9 for producing a medicinal product for the treatment of senile dementia.

## Revendications

1. Composition pharmaceutique solide comprenant :
a) du chlorhydrate de donépézil cristallin de la forme polymorphe I, ayant un modèle de diffraction aux rayons X sur poudre, obtenu en utilisant une irradiation de Cu-K_{α1}, qui a les dix signaux d'intensité les plus forts pour les angles de diffraction 2θ suivants, avec une intensité relative respectivement associée I/I₀
| Angle de diffraction 2θ | Intensité relative I/I₀ |
|---|---|
| [°] | [%] |
| 4,91 | 48,43 |
| 12,64 | 100 |
| 13,07 | 58,57 |
| 13,84 | 22,05 |
| 16,07 | 35,24 |
| 16,87 | 26,21 |
| 21,16 | 71,72 |
| 23,00 | 24,30 |
| 23,90 | 33,38 |
| 24,17 | 20,49 |
et
b) un sel de métal alcalino-terreux de l'acide orthophosphorique.

2. Composition selon la revendication 1, **caractérisée en ce que** le chlorhydrate de donépézil présente une taille moyenne de particules dans une plage de 1 µm à 50 µm.

3. Composition selon l'une des revendications précédentes, **caractérisée en ce que** le sel de métal alcalino-terreux de l'acide phosphorique est l'hydrogénophosphate de calcium.

4. Composition selon l'une des revendications précédentes, **caractérisée en ce que** le rapport molaire entre le sel de métal alcalino-terreux de l'acide phosphorique et le chlorhydrate de donépézil est compris dans une plage de 0,01 à 50.

5. Composition selon l'une des revendications précédentes, **caractérisée en ce que** la fraction de la composition en chlorhydrate de donépézil est comprise entre 1 % en poids et 10 % en poids, sur la base du poids total de la composition.

6. Composition selon l'une des revendications précédentes, **caractérisée en ce que** la composition est directement comprimée.

7. Procédé de fabrication d'une composition selon l'une des revendications 1 à 6, comportant les étapes consistant à :
a) mélanger du chlorhydrate de donépézil cristallin pulvérulent de la forme polymorphe 1 ayant un modèle de diffraction aux rayons X sur poudre, obtenu en utilisant une irradiation de Cu-K_{α1}, qui a les dix signaux d'intensité les plus forts pour les angles de diffraction 2θ suivants, avec une intensité relative respectivement associée I/I₀
| Angle de diffraction 2θ | Intensité relative I/I₀ |
|---|---|
| [°] | [%] |
| 4,91 | 48,43 |
| 12,64 | 100 |
| 13,07 | 58,57 |
| 13,84 | 22,05 |
| 16,07 | 35,24 |
| 16,87 | 26,21 |
| 21,16 | 71,72 |
| 23,00 | 24,30 |
| 23,90 | 33,38 |
| 24,17 | 20,49 |
avec un sel de métal alcalino-terreux pulvérulent de l'acide orthophosphorique, suite à quoi on obtient un mélange pulvérulent chlorhydrate de donépézil / sel d'acide phosphorique de métal alcalino-terreux ; et
b) comprimer le mélange pulvérulent chlorhydrate de donépézil / sel d'acide phosphorique de métal alcalino-terreux pour obtenir un comprimé.

8. Procédé de fabrication d'une composition selon l'une des revendications 1 à 6, comprenant les étapes consistant à :
a) mélanger du chlorhydrate de donépézil cristallin pulvérulent de la forme polymorphe I ayant un modèle de diffraction aux rayons X sur poudre, obtenu en utilisant une irradiation de Cu-K_{α1}, qui a les dix signaux d'intensité les plus forts pour les angles de diffraction 2θ suivants, avec une intensité relative respectivement associée I/I₀
| Angle de diffraction 2θ | Intensité relative I/I₀ |
|---|---|
| [°] | [%] |
| 4,91 | 48,43 |
| 12,64 | 100 |
| 13,07 | 58,57 |
| 13,84 | 22,05 |
| 16,07 | 35,24 |
| 16,87 | 26,21 |
| 21,16 | 71,72 |
| 23,00 | 24,30 |
| 23,90 | 33,38 |
| 24,17 | 20,49 |
avec un sel de métal alcalino-terreux pulvérulent de l'acide orthophosphorique, suite à quoi on obtient un mélange pulvérulent de chlorhydrate de donépézil / sel d'acide phosphorique de métal alcalino-terreux ; et
b) granuler le mélange pulvérulent de chlorhydrate de donépézil / sel d'acide phosphorique de métal alcalino-terreux.

9. Pré-mélange pulvérulent, comprenant
a) un chlorhydrate de donépézil cristallin de la forme polymorphe I ayant un modèle de diffraction aux rayons X sur poudre, obtenu en utilisant une irradiation de Cu-K_{α1}, qui a les dix signaux d'intensité les plus forts pour les angles de diffraction 2θ suivants, avec une intensité relative respectivement associée I/I₀
| Angle de diffraction 2θ | Intensité relative I/I₀ |
|---|---|
| [°] | [%] |
| 4,91 | 48,43 |
| 12,64 | 100 |
| 13,07 | 58,57 |
| 13,84 | 22,05 |
| 16,07 | 35,24 |
| 16,87 | 26,21 |
| 21,16 | 71,72 |
| 23,00 | 24,30 |
| 23,90 | 33,38 |
| 24,17 | 20,49 |
et
b) un sel de métal alcalino-terreux de l'acide orthophosphorique.

10. Utilisation d'une composition selon l'une des revendications 1 à 6 ou d'un pré-mélange selon la revendication 9 pour fabriquer un médicament destiné au traitement de la démence sénile.
